# EUROPEAN PATENT SPECIFICATION

(11) **EP 1 808 077 B1**
(45) Date of publication and mention of the grant of the patent: **11.08.2010**
(21) Application number: 05783371.7
(22) Date of filing: 16.09.2005
(51) Int. Cl.: A61F 2/84, A61M 25/00

(54) **AN ENDOSCOPIC SYSTEM COMPRISING A TREATMENT INSTRUMENT INSTALLATION DEVICE**
ENDOSKOPISCHES SYSTEM MIT EINER VORRICHTUNG ZUR EINLEITUNG EINES BEHANDLUNGSGERÄTES
SYSTÈME ENDOSCOPIQUE AVEC ELEMENT D'INTRODUCTION D'UN MOYEN DE TRAITEMENT

(30) Priority: 24.09.2004 JP 2004277650
(43) Date of publication of application: 18.07.2007
(73) Proprietor: Olympus Corporation, Tokyo 151-0072 (JP)
(72) Inventor: INOUE, Yoshimitsu, 1910001 (JP)
(74) Representative: von Hellfeld, Axel
(86) International application number: PCT/JP2005/017196
(87) International publication number: WO 2006/033309

(56) References cited:
- WO-A-2005/053574
- JP- - 2004 181 230
- JP-A- 5 084 309
- JP-A- 2000 152 985
- US-A- 5 749 918
- US-A1- 2003 088 153
- US-A1- 2004 138 734
- US-B1- 6 287 280
- US-B1- 6 364 901
- US-B1- 6 537 266

## Description

### BACKGROUND OF THE INVENTION

### 1. Field of the Invention

The present invention relates to a system composed of an endoscope, a treatment instrument installation device and a treatment instrument, as is known from US-A-5,749,918.

### 2. Description of the Related Art

When stenosis develops at the bile duct or the like inside the human body, it is well known to secure a passage of the bile duct or the like by inserting an elongated tubular stent into the bile duct or the like and further inserting through the stent inside of the stenotic area. At the time of inserting the stent, the stent is provided on an elongated guide member, and a guide member is inserted through a conduit of an endoscope into the inside of the bile duct or the like from the tip of the endoscope and then the stent is pushed out of the guide member. The stent is provided with flaps and by opening these flaps the stent is prevented from being dislodged from the stenosis area.

Conventionally, at the time of inserting the stent, a stent installation member to guide the stent into the conduit of endoscope has been used. The stent installation member has substantially the same inner diameter as the opening of the insertion slot of the conduit and the stent installation member is made so that the stent provided inside of the stent installation member is guided into the conduit if the stent installation member is pressed against the surroundings of the insertion slot of the conduit.

Because the flap provided on the proximal side of the stent is made to open towards the distal end of the stent, it is possible for the flap on the proximal side to bent due to interference with the insertion slot of the conduit at the time of inserting the stent. Because of this, an invention such as Patent Document 1 is disclosed that enables the tip of the stent installation member to be inserted into the insertion slot of the conduit. The stent installation member has a length to completely cover the flap provided on the proximal side of the stent and therefore the flap on the proximal side is kept covered by the stent installation member and inserted into the insertion slot of the conduit.

However, the conduit of the endoscope is divaricated into branches to perform insufflations or the like and the diameter of the conduit is larger at the bifurcation, and therefore, when the stent is passing through the bifurcation, the flaps may open because the diameter of the conduit is larger there. If the flap opens at the bifurcation, it may be bent when the flap is further inserted into the conduit which has a diameter smaller than that of the bifurcation. Therefore, even if the flaps are protected at the time of insertion at the insertion slot of the conduit, if the conduit has an area with a larger diameter than that of the insertion slot, the flap may be bent or the like. A bent flap as mentioned above tends to be caught by a support pillar provided at the distal end of the endoscope making it difficult to push the stent out of the endoscope. Apart from the case using the stent, in the case of using a guide wire, papilotomy knife or angiographic catheter, it is known that if there is bending at the tip of a sheath, it is easy to become caught at the bifurcation. This invention was made considering the above-mentioned situations and its purpose is to be able to place the stent within the human body without fail.

It is on object of the present invention to provide a system as outlined above allowing a smooth and secure installation of a treatment instrument.

The system according to the invention is described in claim 1. Preferred embodiments are described in the dependent claims.

According to this installation member a conduit which has a smaller inner diameter than that of the connection part is created by the main body through which the first treatment instrument is inserted.

Preferably, the main body of the above mentioned treatment instrument installation member is provided with a regulative part on its proximal end which regulates the installation amount of the main body into the first conduit.

According to this installation member, an insertion into the correct location can be achieved by the installation amount being regulated by the regulative part.

The stent delivery catheter comprises a guide member inserted into the first conduit and the main conduit from the insertion slot for the first treatment instrument provided at the operation area of the endoscope, a stent which is movable forward and backward which is provided on the guide member, a pushing member which is movable forward and backward which is provided on the guide member to push the stent out of the guide member to be placed in the human body, and a stent installation member which corresponds the above-mentioned treatment instrument installation member which is movable forward and backward which is provided on the pushing member and on the stent.

According to the stent delivery catheter the insertion of the stent can be performed with a part of the opening of the connection part blocked by the stent installation member, and therefore the bending of the flap of the stent, which is caused by the opening of the flap, is prevented when the stent is passing through the connection part.

Preferably, the stent of the above mentioned stent delivery catheter is provided with flaps which regulate the movement of the stent, and the stent delivery catheter is provided with a protective case which covers the stent with its flaps open and with the stent provided on the guide member.

The flaps of the stent are prevented from being bent during storage by being provided with a protection case.

According to the system of the present invention, the stent can be inserted in the correct condition because the flap of the stent is prevented from being bent at the connection part when the stent is inserted into the human body.

### BRIEF DESCRIPTION OF THE DRAWINGS

FIG 1 is an overall view of an endoscope treatment system including a stent delivery catheter according to an embodiment of the present invention.
FIG. 2 is a cross section of a partially enlarged side view of the endoscope treatment system of FIG. 1.
FIG. 3 is an overall view showing a structure of the stent delivery catheter.
FIG. 4 is a view explaining a usage of the stent delivery catheter.
FIG. 5 is a cross section of a partially enlarged side view of the endoscope treatment system of FIG. 1, showing a stent inserted into a main conduit.
FIG. 6 is a view showing the stent inserted into a living body.
FIG. 7 is a view showing the stent.
FIG. 8 is a view showing the stent delivery catheter and its protective case.
FIG. 9 is a side view of the protective case.
FIG. 10 is a side view of the stent installation member.
FIG. 11 is a view showing the installation of the stent.
FIG. 12 is a side view showing the stent installation member inserted into the endoscope.
FIG. 13 is a side view of the stent installation member.
FIG. 14 is a side view showing the stent installation member inserted into the endoscope.
FIG. 15 is a side view of the stent installation member.
FIG. 16 is a side view showing a stent installation member inserted into the endoscope.
FIG. 17 is a view of a regulative member seen from the conduit B-B toward the direction of the arrow in FIG. 16.

### DETAILED DESCRIPTION OF THE INVENTION

The best mode for carrying out the invention of the present application is explained by referring to the drawings.

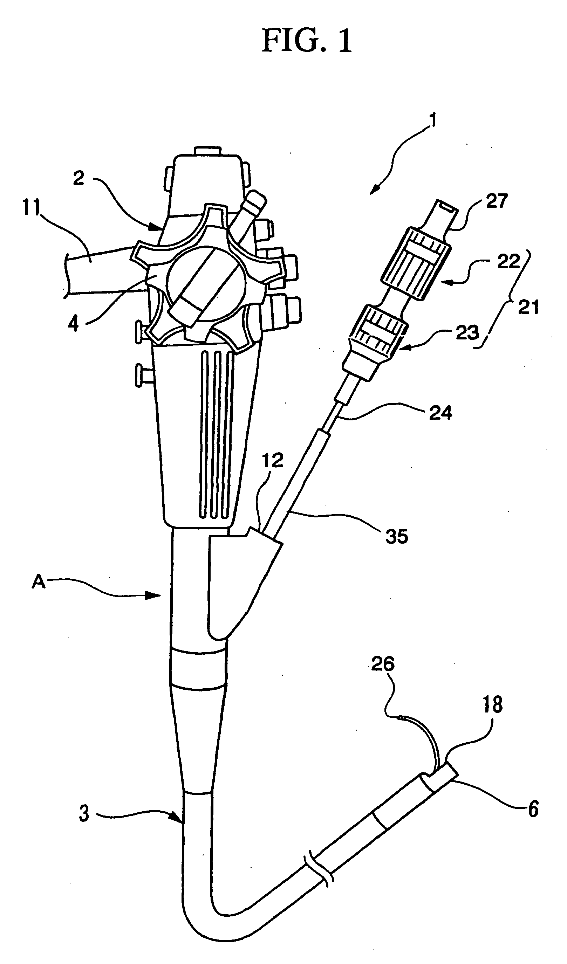

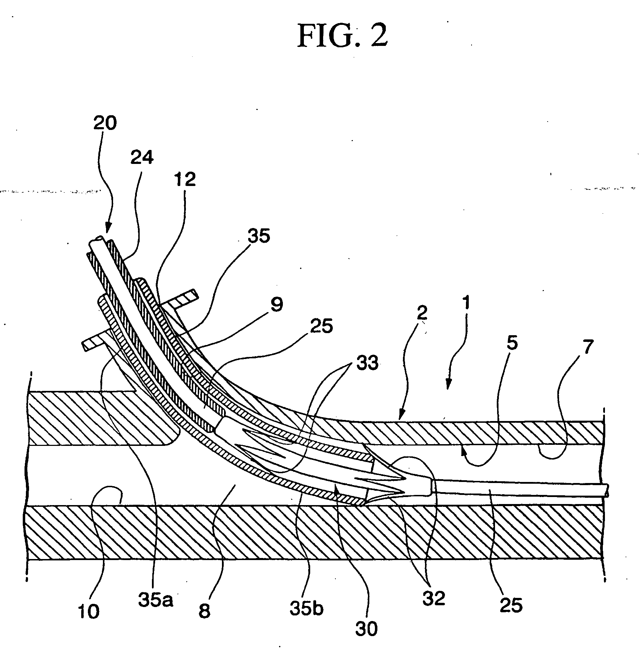

FIG. 1 is a view showing a stent delivery catheter according to a first embodiment of the present invention inserted through an endoscope and FIG. 2 is an enlarged view of the cross section of part A of FIG. 1.

As shown in FIGS. 1 and 2, an endoscope 1 has an endoscope operation part 2 that an operator operates and, on the tip of the endoscope operation part 2, an endoscope insertion part 3 is provided to be inserted into the human body. The endoscope insertion part 3 is flexible and can be bent by a knob 4 of the endoscope operation part 2. A treatment instrument insertion channel 5, which is a conduit system, is provided inside of the endoscope 1. A distal end 6 of the treatment instrument insertion channel 5 has an opening on the side of the distal end 6 of the endoscope insertion part 3 and communicates with the endoscope operation part 2 through the endoscope insertion part 3.

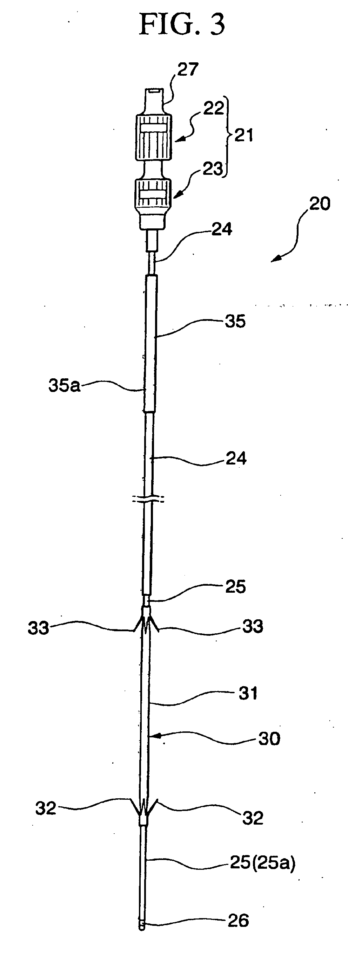

The treatment instrument insertion channel 5 comprises a main conduit 7 which communicates with the distal end 6 of the endoscope insertion part 3 and a first conduit 9, and is divaricated to a second conduit 10 at a bifurcation 8 which is a connection part. The second conduit 10 extends along the axis of the main conduit 7 and communicates with a liquid supply conduit and air supply conduit which are both not shown, and can supply normal saline as a second treatment instrument. The liquid supply conduit or the air supply conduit are communicate with a liquid supply apparatus or an air supply apparatus provided outside via a universal cord 11 which extends from the side of the endoscope operation part 2 as shown in FIG. 1. The first conduit 9 is extended to an insertion slot 12 provided on the side of the endoscope operation part 2. A stent delivery catheter 20 is inserted as the first treatment instrument into the insertion slot 12. As shown in FIGS. 1 and 3, the stent delivery catheter 20 has an operation part
21 with which an operator operates. An operation part 21 comprises a mouth ring 22 for a guide catheter and a mouth ring 23 for a pusher tube which is removably provided on the mouth ring 22 for a guide catheter. The mouth ring 23 for a pusher tube is provided with a pusher tube 24 on the distal end of the mouth ring 23 for a pusher tube. The pusher tube 24 comprises a flexible elongated tubular member. A guide catheter 25, which is a guide member, is movably inserted forward and backward through the pusher tube 24. The guide catheter 25 comprises a flexible member and is provided with lumens and a marker 26 for X-ray imaging which is buried on the distal end of the guide catheter 25. The lumens, which is passing through inside of the mouth ring 23 for a pusher tube, and communicate with a connection part 27 provided on the proximal end of the mouth ring 22 for a guide catheter. The connection part 27 comprises a luer-lock or the like and can communicate with syringes or the like which are not shown in figures.

The guide catheter 25 is longer than the pusher tube 24 and the distal end of the guide catheter 25 is exposed from the distal end of the pusher tube 24 when the mouth ring 22 for a guide catheter and the mouth ring 23 for a pusher tube are connected. A stent 30 is movably provided forward and backward around the exposing distal end 25a.

The stent 30 is provided with a tubular main stent body 31. The inner diameter of a main stent body 31 is substantially the same as the outer diameter of the guide catheter 25. The outer diameter of the main stent body 31 is smaller than the inner diameters of those of the main conduit 7 or the bifurcation 8 or the first conduit 9. A flap 32 is provided on the distal end of the stent 30. Four flaps 32 are provided on the circumference of the stent main body 31 and each of the flaps has a 90-degree interval with adjacent flaps, and when a pressure is not applied they are made to open toward the proximal end of the stent 30 by lifting the tip of the flap which is closer to the proximal end. And when a prescribed pressure is applied they are capable of changing their shapes to close themselves. Each of the four flaps 32 is made so as to gradually reduce its width from the proximal end, which is connected to the main stent body 31, to its distal end. Four flaps 33 are provided on the proximal end of the stent 30. The flaps 33 have the same structure as the flaps 32 except that when a pressure is not applied they are made to open toward the distal end of the stent 30 by lifting the four tips of the flap closer to the distal end.

Further, a stent installation member 35 is movably provided forward and backward around the pusher tube 24. The stent installation member 35 comprises a tubular main body 35a and the inner diameter of the tubular main body 35a is larger than the outer diameter of the pusher tube 24. The inner diameter of the stent installation member 35 is larger than the outer diameter of the stent 30 in a state where both the flaps 32 and 33 are closed. The outer diameter of the stent installation member 35 is smaller than the inner diameter of the first conduit 9 which is located closer to the endoscope 1 and the inner diameter of the bifurcation 8. As explained in detail later, the length of the stent installation member 35 is longer than the total length of the first conduit 9 and is shorter than the pusher tube 24 and more preferably, it is approximately 90 mm.

The stent installation member 35 as described above is made of fluorine resin (including PTFE, PFT, FEP or the like) or polyethylene resin or the like. The reason for this is because the stent installation member 35 should be thin, rigid and smooth enough to enable the insertion of the stent installation member 35 into the first conduit 9. And in the stent installation member 35, at least a distal end 35b thereof may be made to be deformable. A structure which enables the distal end 35b of the stent installation member 35 to be deformable is achieved by making, for example, the whole stent installation member 35 or the distal end 35b of the stent installation member 35 with a flexible material or to make the distal end 35b of the stent installation member 35 thinner than other parts or to provide slits on the distal end 35b of the stent installation member 35 along the longitudinal direction or the like. This kind of distal end 35b of the stent installation member 35 acts as a blocking member by reducing the area through which the stent 30 passes at the bifurcation 8.

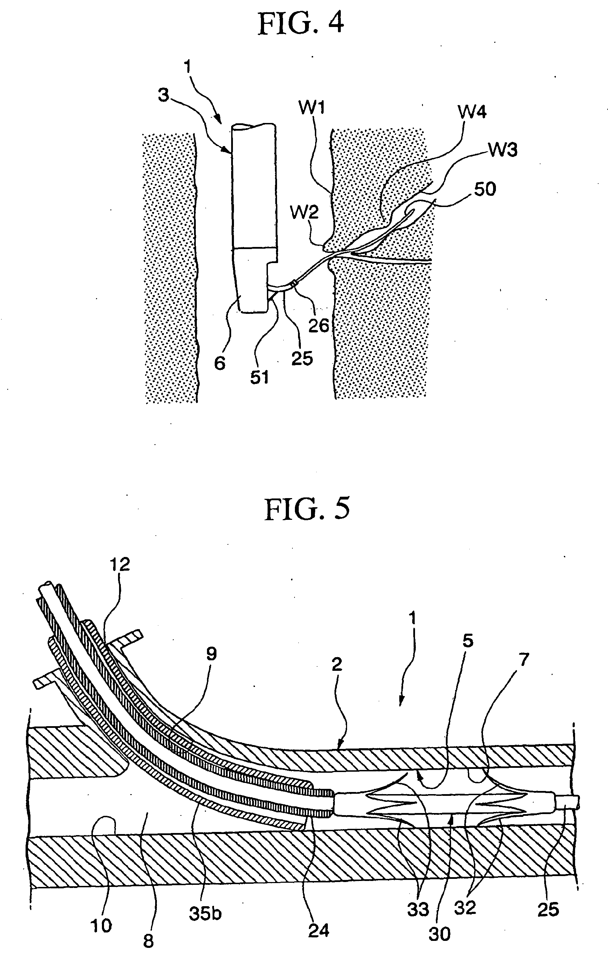

Next, the operation of this embodiment of present invention is explained. The explanation is made referring to an example shown in FIG. 4 which shows a case where the stent 30 is inserted into a bile duct W3 from a duodenal papilla W2 of the duodenum W1 of the patient. In FIG. 4, a guide wire 50 is inserted into the bile duct W3. The guide wire 50 is pre-inserted from the duodenal papilla W2 by using an angiographic tube or the like which is not shown in figures. And when the angiographic tube is pulled out, the distal end of the guide wire 50 remains inside of the bile duct W3 and at the same time the proximal end of the guide wire 50 is pulled outside the body passing through the treatment instrument insertion channel 5 of the endoscope 1.

In the case of inserting the stent 30 as shown in FIG. 3, the mouth ring 22 for a guide catheter and the mouth ring 23 for a pusher tube are connected first and then the pusher tube 24 and the guide catheter 25 are connected via the operation part 21. And then the stent installation member 35 is slid toward the distal end of the guide catheter 25 and the flap 33 provided on the proximal end of the stent 30 is covered with the stent installation member 35.

Next, the proximal end of the guide wire 50 is inserted into the lumen from the distal end of the guide catheter 25 and pulled out from the connection part 27 of the mouth ring 22 for a guide catheter of the operation part 21 and then the guide catheter 25 is inserted into the first conduit 9 from the insertion slot 12 of the endoscope 1. Because the outer diameter of the guide catheter 25 is much smaller than the inner diameter of the first conduit 9 and the bifurcation 8, the guide catheter 25 is bent at the bifurcation 8 and led along the main conduit 7 and directed toward the distal end of the main conduit 7. As the guide catheter 25 is directed, the stent 30 and the stent installation member 35 which is provided around the proximal end of the stent 30 are also inserted into the first conduit 9. (Stent installation member insertion process)

The stent 30 is inserted into the first conduit 9 while the flap 32 provided on the distall end of the stent 30 is closed. And the stent 30 is bent along the guide catheter 25 at the bifurcation 8 when the stent 30 proceeds from the first conduit 9 into the main conduit 7. Because the apparent inner diameter is increased at the bifurcation 8, each flap 32 opens temporarily at the bifurcation 8 and closes again when the stent is inserted into the main conduit 7. Because the flaps 32 are made to open by lifting the four tips of the flap closer to the proximal end of the stent 30, the flaps 32 are inserted into the main conduit 7 without being bent as shown in FIG. 2.

The stent installation member 35, which is inserted into the bifurcation 8 following the distal end of the stent 30, is inserted into the main conduit 7 with the distal end of the stent installation member 35 being bent at the bifurcation 8. However, the stent installation member 35, which has substantially the same outer diameter as the inner diameter of the first conduit 9 and has rigidity as a whole, stops as if it fits into the first conduit 9 and the main conduit 7. In this state, by further inserting the guide catheter 25 into the treatment instrument insertion channel 5, the stent installation member 35 remains stationary and the stent 30 moves forward as it is pushed by the pusher tube 24. As a result, as shown in FIG. 5, the proximal end of the stent 30 is pushed out of the distal end of the stent installation member 35. Because the flaps 33 are pushed out of the stent installation member 35 after passing through the bifurcation 8, which has an increased inner diameter, even if the flap 33 opens inside of the main conduit 7, the flap 33 is not bent.

And if the guide catheter 25 reaches the distal end of the endoscope 1, as shown in FIG. 4, the position of the distal end of the guide catheter 25 is adjusted to the position of the duodenal papilla W2 by raising a support pillar 51 of the distal end 6 of the endoscope 1. When the positioning is completed, the guide catheter 25 is further pushed and its distal end is inserted inside of the bile duct W3. (Stent installation process)

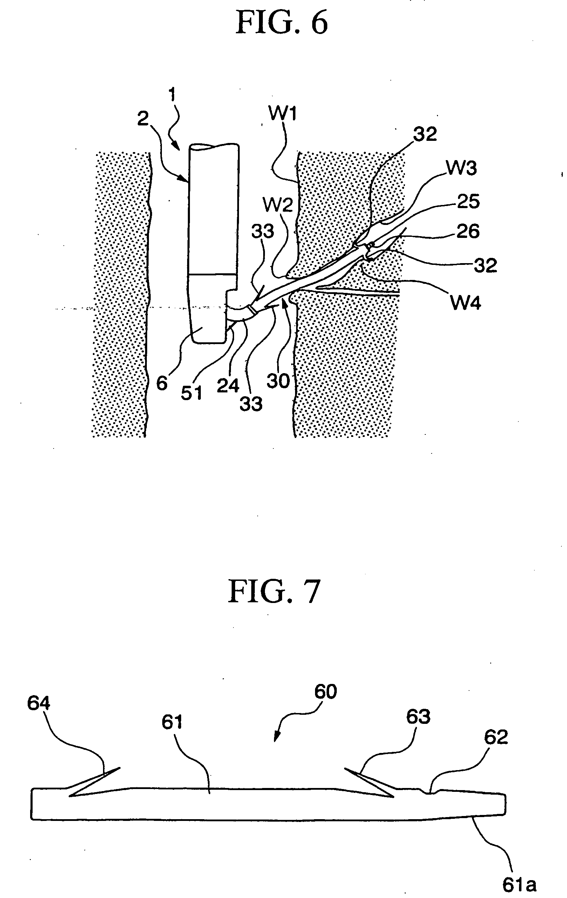

When the marker 26 is inserted so much that it passes over a stenosis W4, the guide catheter 25 is fixed and the mouth rings 22 for a guide catheter, 23 for a pusher tube of the operation part 21 are separated each other and the pusher tube 24 is moved forward along the guide catheter 25. When the pusher tube 24 moves forward, the distal end of the pusher tube 24 pushes the stent 30 toward the distal end of the guide catheter 25. Because of this, as shown in FIG. 6, the distal end of the stent 30 is passed through the duodenal papilla W2 and inserted into the bile duct W3 and is located in an area deeper than the stenosis W4. As the flaps 32 are open toward the proximal end of the stent at this time, they are closed when they pass through the duodenal papilla W2, however, they open again in the area deeper than the stenosis W4 inside of the bile duct W3. The flaps 33 open toward the distal end of the stent 30 inside of the duodenum W1. And when the guide catheter 25 is pulled out in a state that the pusher tube 24 is fixed relative to the endoscope 1, then the stent 30 is installed. As a result, the bile duct W3 and the duodenum W 1 communicate through a through-hole provided inside of the stent main body 31. A movement toward the bile duct W3 is regulated by the flap 33 and the movement of the stent 30 toward the duodenum W1 is regulated by the flap 32.

According to this embodiment of the present invention, since the stent installation member 35 is inserted into the treatment instrument insertion channel 5 and the distal end of the stent installation member 35 reaches beyond the bifurcation 8 to the main conduit 7, it is possible to reduce the inner diameter of the space where the stent 30 passes through in the vicinity of the bifurcation 8 and the flaps 33 of the stent 30 are prevented from being open in the bifurcation 8. If the stent installation member 35 is not provided, when the stent proceeds from the bifurcation 8 into the main conduit 7, the flaps 33 which open toward the distal end of the stent 30 tend to be bent because the inner diameter is reduced, however, in this embodiment of the present invention this problem is prevented. Therefore, during the insertion of the stent 30, the bent flap is prevented from getting stuck by the support pillar 51. Furthermore, when the stent 30 is placed in the human body, the stent 30 is not easily dislodged because of the correct functions of the flaps 32 and 33. Because the stent installation member 35 has such a rigidity to be inserted into the first conduit 9 and the distal end of the stent installation member 35 is made to change its shape along the bifurcation 8 and the main conduit 7, deformation or the like of the flaps 33 are prevented by simply inserting the stent installation member 35 into the endoscope 1 until it stops.

Furthermore, because the stent 30 and the stent installation member 35 are preliminarily provided around the guide catheter 25 and pusher tube 24, the stent delivery catheter 20 contributes to reducing the burden of placing the stent 30 during surgery and lessening the surgery time.

The stent used as one of the embodiments of the present invention can be as follows.

As shown in FIG. 7, a stent 60 comprises a main body 61 which is a tubular blocking part provided with a through-hole. The circumference of a distal end 61a of the main body 61 is tapered so as to reduce the outer diameter of the distal end 61 a of the main body 61 toward its distal end. On the more proximal side than the chamfering part of the distal end 61a of the main body 61, a lateral hole 62 is provided which has an opening on the lateral face of the main body 61 which connects with the through-hole of the main body 61 in a perpendicular direction. On the more proximal side than the lateral hole 62, a flap 63 is provided. The flap 63 is made so that a part of the main body 61 is peeled from the proximal end to the distal end of the stent 60 by lifting the distal end of the flap and the width of the flap 63 is reduced toward its distal end from the proximal end which is connected to the main body 61, and the flap 63 is made so as to be able to change shape by a force applied from outside. On the proximal end of the main body 61, a flap 64 is provided and its structure is the same as that of the flap 63 except that the distal end of the flap 64 is lifted toward the distal end of the main body 61

The stent 60 has a longer distance from its distal end and to the flap 63 than the conventional stent and has a shorter distance from its proximal end to the flap 64 than the conventional stents. By keeping the distance on the distal end of the main body longer, the interference between the surface of the lumens like the bile duct W3 or the like and the distal end of the stent 60 is minimized. And by keeping the distance on the proximal end of the main stent body shorter, disruption of the flow of food or the like inside of the duodenum W1 is reduced because it keeps the amount of projection of the proximal end of the stent 60 out to the duodenum W1 small when the proximal end of the stent 60 is disposed inside of the duodenum W1 for example. Considering the insertion of the stent 60 into the bile duct W3 as above for example, preferably, the distance between the distal end 61 a of the stent 60 to the flap 63 is 20 mm and the distance between the proximal end of the stent 60 to the flap 64 is 12 mm.

Because the distal end 61 a of the stent 60 is tapered, it is possible to smoothly insert it into the duodenal papilla W2 or the like thereby reducing discomfort to the patient. Because the distal side of the stent 60 which is inserted into the bile duct W3 or the like is made longer than the conventional stents, damage or the like to the mucous membrane on the tube wall is reduced because the distal end 60a of the stent 60 does not easily become stuck in the wall surface. Furthermore, because the proximal end of the stent 60 is shorter, the flow of food or the like is kept smoother and an uncomfortable feeling in the patient is reduced. As with the previous embodiment of the stent, it is preferable to provide the stent 60 around the guide catheter 25 preliminarily. As a result, it is possible to avoid setting the direction of the stent 60 reversely which has a tapered distal end 61 a or has a different length on the distal side 60a and the proximal side.

Furthermore, this embodiment of the present invention may be placed in the following case.

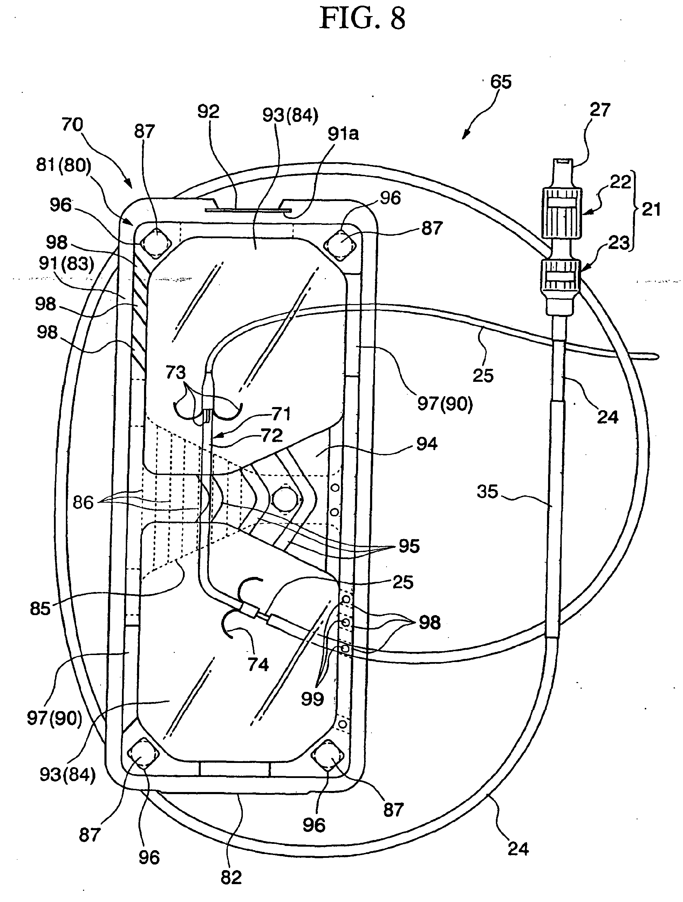

As shown in FIG. 8, a stent delivery catheter 65 is provided with a protective case 70. In this embodiment of the present invention, a stent 71 is provided around the guide catheter 25. The stent 71 comprises a bent main body 72 and four flaps 73 which are provided around the distal end of the main body 72 at equal intervals in the circumferential direction. Each of the flaps 73 is curved so that it opens toward the proximal end of the stent. In a similar way, on the proximal side, four flaps 74 are provided at equal intervals in the circumferential direction and each of the flaps 74 is curved so that it opens toward the distal end of the stent. This kind of stent 71 is used in a case, for example, to promote the expulsion of bile in the stenotic area when a stenosis developes in a bile duct.

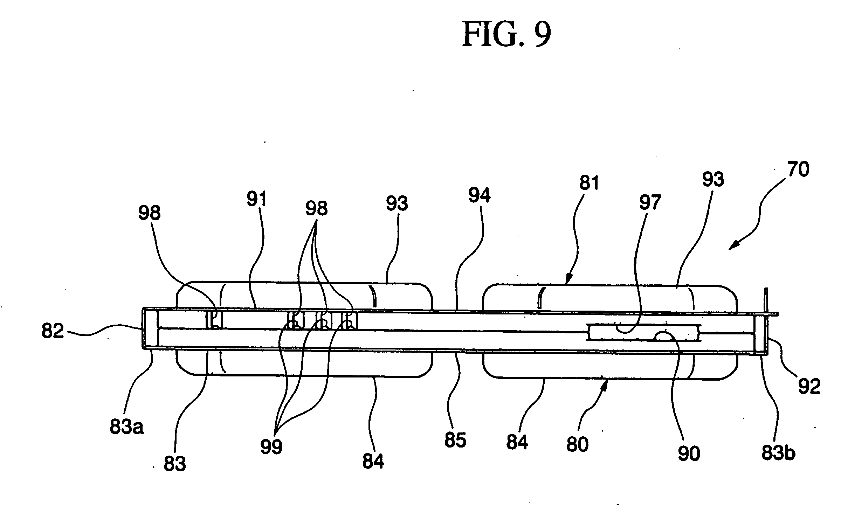

As shown in FIGS. 8 and 9, the protective case 70 is constructed so that a basement 80 and a lid 81 are connected via a connection part 82 and when overlapping the basement 80 with the lid 81 by bending the connection part 82, it is possible to house the stent 71, a part of the guide catheter 25 and a part of the pusher tube 24 in the space created thereinside.

As shown in FIG. 9, the basement 80 comprises a housing part 84, except a flange 83 which is the surroundings of the basement 80, and a rib 85 which keeps the strength provided on substantially the center in the longitudinal direction on the basement 80. As shown in FIG. 8, the rib 85 is provided with a plurality of grooves 86 along the longitudinal direction of the rib 85 which is also the direction of the narrow side of the basement 80. These grooves 86 are made to removably hold the straight-conduit portion of the stent 71 and the shapes of the grooves are adjusted to the diameter or the shape of the stent 71. The flange 83 is provided with a plurality of convexes 87 which fit together with the lid 81 on the four corners.

On one lateral side of the longitudinal direction of the basement 80, a slit 90 is provided through which the guide catheter 25 passes. The width of the slit 90 is much bigger than the outer diameter of the guide catheter 25. On the flange 83 of the basement 80, the connection part 82 is provided on one fringe 83a which is in the direction of narrow side of the basement 80 and the basement 80 and the lid 81 is formed integrally via the connection part 82. On another fringe 83b, a tongue 92 is provided on the extension of the fringe 83b which is to be held with a flange 91 of the lid 81.

The lid 81 is comprised of a housing part 93, except for the flange 91 which is in the surroundings of the basement 81, and a rib 94 on substantially the center in the longitudinal direction on the lid 81. The rib 94 is provided with a plurality of grooves 95 which are made to removably hold the straight-conduit portion of the stent 71 along the longitudinal direction of the rib 94. These grooves 95 are made in a bent shape to hold the bent portion of the stent 71. The flange 91, which is overlapped with the flange 83 of the basement 80, is provided with a plurality of concaves 96 on the four corners which fit together with the convexes 87 of the flange 83.

Furthermore, on one lateral side of the longitudinal direction of the lid 81, a slit 97 is provided through which the guide catheter 25 passes. The slit 97 is formed on a position which overlaps with the slit 90 and formed in the same shape as the slit 90 and the slit 97 creates a space with the slit 90 through which the guide catheter 25 is inserted. A plurality of slits 98 are provided in a symmetrical position against the slit 90 across the rib 94 on one lateral side of the lid 81. Each slit 98, which is to be inserted through by the correctly positioned pusher tube 24, has a width substantially the same as the outer diameter of the pusher tube 24 and a plurality of the slits 98 are provided on the lid 81 along its longitudinal direction. Each slit 98 is slanted off in the longitudinal direction of the lid 81. In order to prevent the pusher tube 24 from deviating, a plurality of swells 99 is provided at a location symmetrical against each of the slits 98. As shown in FIG. 8, on the flange 91 of the lid 81, an engagement 91a is provided to hold the tongue 92.

This stent delivery catheter 65 which is covered by the protective case 70 is stored in a sterile pack. The height of the housing parts 84 or 93 of the protective case 70 is much higher than the projected amount of the flap 73 or 74 and the housing part 84 or 93 has sufficient resistance against an external force. Therefore, in this state, the flap 73 or 74 of the stent 71 is kept open without being interfered with by the wall of the protective case 70 and their deformation is restrained. And the deviation of the stent 71 is also restrained because it is held by the groove 86.

When the stent delivery catheter 20 is used, it is taken out of the sterile pack and then the protective case 70 is opened to remove the pusher tube 24 out of the slit 98 and pick up the stent 71 out of the groove 86. Subsequent method of usage is the same as that of the first embodiment of the present invention.

As described above, because it is possible to store and use the stent delivery catheter 65 as one integrated unit, handling thereof becomes easy. And because the stent 71 is held by the protective case 70, deformation or the like of the flap 73 or 74 more than necessary can be avoided.

Furthermore, by providing the slit 98 on the protective case 70, it is possible to hold the pusher tube 24 in a fixed state and this protects the stent 71 from being subjected to unexpected stresses. Because the guide catheter 25 passes through the wide slit 90 or 97, even when the stent 71 is held by any of the grooves 86, unexpected forces are not applied to the guide catheter 25.

When the stent 71 is held by the groove 95 on the lid 81, the guide catheter 25 and the pusher tube 24 are inserted through the slits 90, 97 and 98 provided on the opposite side of the stent delivery catheter 65 shown in FIG. 8. And the stent installation member 35 may be held by either of the groove 86 or the groove 95.

Next, the second embodiment of the present invention is explained by referring to the figures. The same symbols are provided for the structural members common to the first embodiment of the present invention. Explanation is omitted of the structural members already explained above.

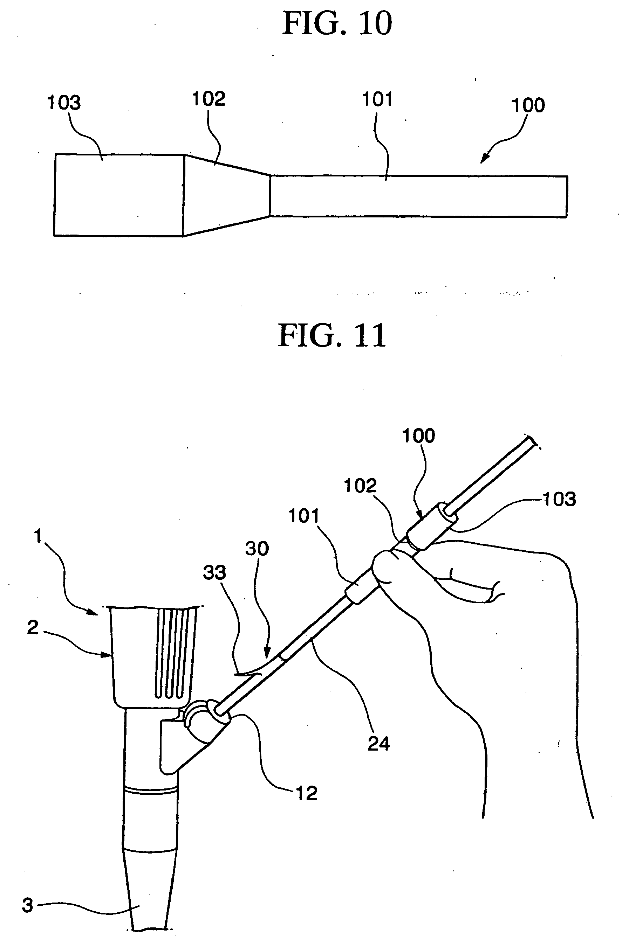

As shown in FIG. 10, a stent installation member 100 comprises a tubular main body 101 which functions as a blocking part and a tapered transition part 102 which functions as a regulative part on the proximal side of the main body 101. The outer diameter of a proximal end part 103 of the stent installation member 100 is larger than that of the main body 101 due to the transition part 102. The main body 101, transition part 102 and the proximal end part 103 of the stent installation member 100 are integrally formed and they are shaped in a hollow tubular shape. The main body 101 is the blocking part provided with an outer diameter so that it can be inserted into the first conduit 9 and further into the bifurcation 7 from the insertion slot 12. The inner diameter of the main body 101 is made so that the stent 30 can be inserted in a state such that the flaps 32 and 33 are closed. The length of the main body 101 is longer than the route from the insertion slot 12 to the bifurcation 8 and is shorter than the pusher tube 24 and is preferably, for example, 60 mm. And the proximal end part 103 is provided with an outer diameter larger than the insertion slot 12 and is provided with an inner diameter so that the stent 30 can be inserted. The transition part 102 is made, for example, by thermoforming a tube, by covering a heat-shrinkable tube so as to have a step, or by connecting different tubes with different outer diameters.

The function of the stent installation member 100 will be explained.

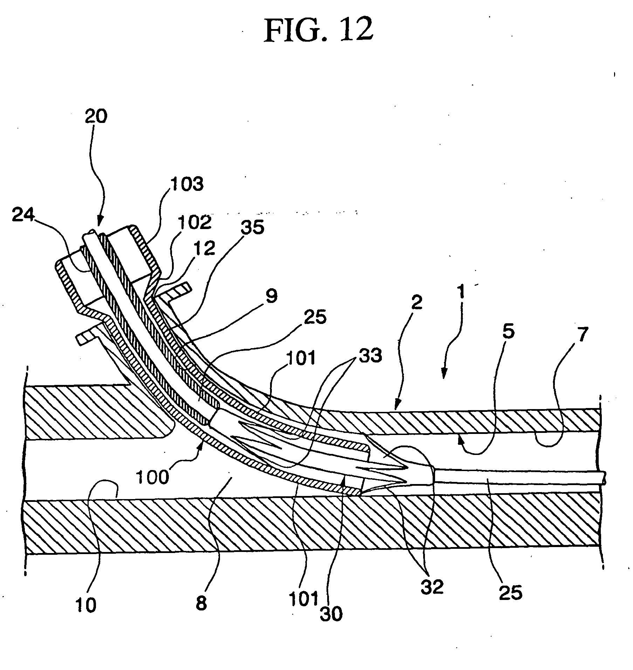

As shown in FIG. 11, the distal end of the guide catheter 25 and the distal end of the stent 30 are inserted into the treatment instrument insertion channel 5 from the insertion slot 12 of the endoscope 1. Before the flap 33 on the proximal side of the stent 30 is inserted from the insertion slot 12, the stent installation member 100 is inserted from the insertion slot 12. As shown in FIG. 12, the stent installation member 100 is inserted until the transition part 102 reaches the insertion slot 12. At this time, the distal end of the main body 101 reaches into the main conduit 7 passing through the first conduit 9 and the bifurcation 8. In this state, when the stent 30 is further inserted, the flap 33 on the proximal side of the stent 30 is covered inside of the stent installation member 100 and the stent reaches the main conduit 7 through the bifurcation 8.

In this manner, when the stent installation member 100 is used, by the transition part 102 being caught by the insertion slot 12, the insertion amount of the stent installation member 100 is regulated so as to be most suitable for the installation of the stent 30, accordingly, the stent installation member 100 is prevented from being inserted more than necessary into the endoscope 1. Other functions and effects are the same as those of the first embodiment of the present invention as described above.

In this embodiment of the present invention, the following stent installation member can be also used.

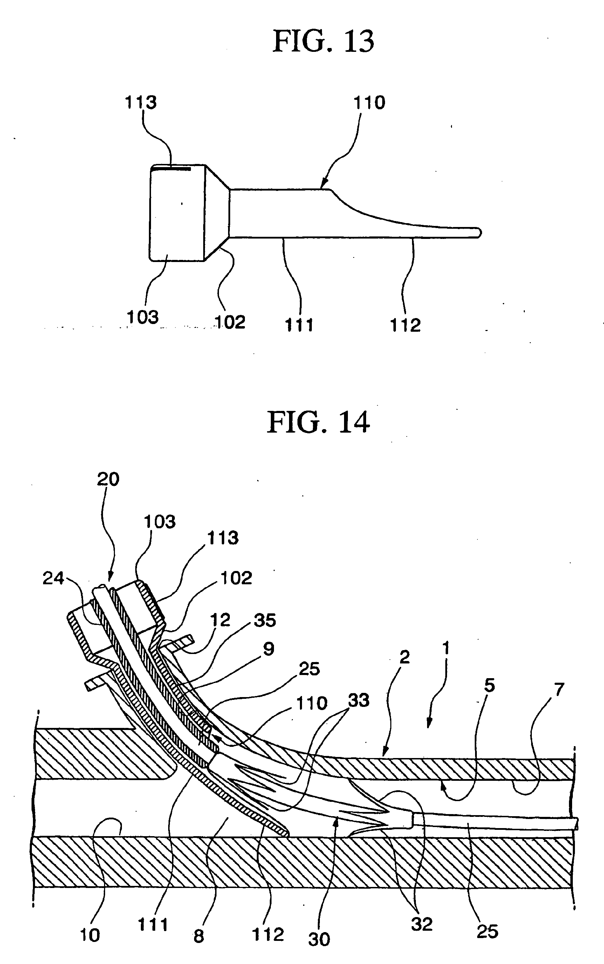

As shown in FIG. 13, a stent installation member 110 comprises a tubular main body 111 and a proximal part 103 provided on the proximal end of the main body 111 which has a larger diameter by being connected to the transition part 102. On the extension of the distal end of the main body 111, a blocking member 112 is provided.

The blocking member 112 comprises a part of the circumference of the main body 111 extending along the axis of the main body. The inner diameter of the main body 111 is made so that it can house the stent 30 in a state such that the flaps 32 and 33 are closed, and the outer diameter of the main body 111 is smaller than the diameter of the first conduit 9. The length of the main body 111 is shorter than the total length of the first conduit 9, however, as a whole stent installation member 110, it is long enough to reach the main conduit 7 through the bifurcation 8 from the insertion slot 12. The length of the main body 111 is preferably, for example, approximately 30 mm and the length of the blocking part 112 in this case is approximately 30 mm.

A marker 113 is provided on the proximal part 103 of the stent installation member 110 so that the position where the blocking part 112 is extended can be identified in the cirumferential direction.

The marker 113 is provided at a position shifted by 180 degrees in the circumferential direction from the extended part of the blocking part 112. In other words, on the stent installation member 110 a part of which is slit in the circumferential direction, a marker 113 is provided on a corresponding part of the stent installation member 110 which is slit.

The stent installation member 110 is inserted into the insertion slot 12 when the stent 30 is inserted. The stent installation member 110 is inserted so that the marker 113 of the proximal part 103 is directed to the insertion part 3 of the endoscope, that is, to the downside in FIG. 1, and the stent installation member is inserted until the transition part 102 contacts the insertion slot 12. As shown in FIG. 14, when the stent installation member 110 is inserted in this direction, it is inserted so that the blocking part 112 heads upward at the bifurcation 8 so as to block the opening communicating with the second conduit 10. When the stent 30 is sent toward the main conduit 7, in this state, one piece of the flap 33 which is provided on the proximal side of the stent 30 and facing the opening communicating with the second conduit 10 maintains contact with the blocking part 112 and is guided into the main conduit 7. As a result, the flap 33 does not open in the bifurcation 8 and is guided into the main conduit 7.

In the case that the stent installation member 110 is used, because the flap 33 does not open in the bifurcation 8, the flap 33 is prevented from being bent or the like. And the stent installation member 110 is made so that the blocking part 112 is the extended part of the main body 111, the stent installation member 110 is easy to transform along the conduit and therefore increases workability. By providing the transition part 102, it is possible to regulate the insertion amount of the stent installation member 110 and therefore it is possible to insert the stent 30 without fail. In this case, by providing the marker 113 on the proximal end part 103, it is easier to check the position of the blocking part 112 visually and therefore it is possible to position the blocking part 112 at the opening communicating with the second conduit 10 in the bifurcation 8 without fail.

Next, a third embodiment of the present invention is explained referring to the figures. The same symbols are provided for the structural members which are common to the above-described embodiment of the present invention. Explanation is omitted of the structural members already explained above.

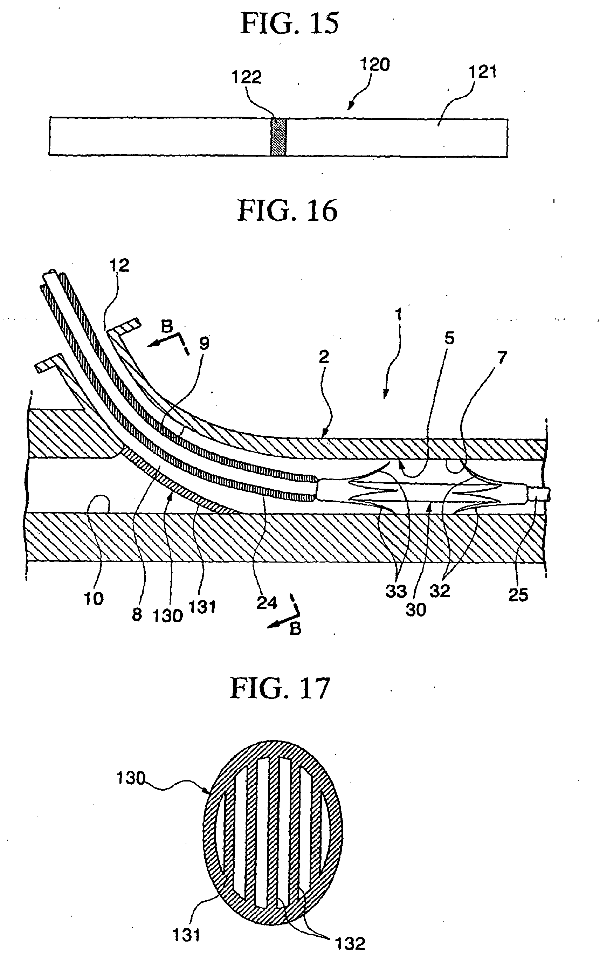

As shown in FIG. 15, a stent installation member 120 is provided with a main body 121 which is a tubular blocking part and on a circumferential surface from the distal end to the proximal end of the stent installation member a marker 122, which is an annular identification member, is provided. The marker 122 is provided at a position which corresponds to the length from a slot for clamps 12 to the main conduit 7 through the bifurcation 8 and it is provided approximately 60 mm from the distal end of the stent installation member 120 for example. The inner diameter, outer diameter and the length of the stent installation member 120 are the same as those of the first embodiment of the present invention.

A function of the stent installation member 120 will be explained.

The installation member 120 is inserted into the slot for clamps 12 until the marker 122 reaches the slot for clamps 12. At this time, the distal end of the main body 121 reaches the main conduit 7 through the bifurcation 8. And when the stent 30 is guided into the main conduit 7 through the inside of the stent installation member 120, the stent 30 is installed with its flap 33 closed.

According to the stent installation member 120, by visually checking the marker 122, it is possible to identify the inserted amount of the stent installation member 120, and the stent installation member 120 can be prevented from being inserted too much into the endoscope 1. Other functions and effects of the stent installation member 120 are the same as those of the first embodiment of the present invention.

An embodiment of the marker 122 may be provided by painting the main body 121 in a different color. In particular, the distal side and proximal side from the position corresponding to the marker 122 is identified in different colors.

Next, an example is explained referring to the figures. The same symbols are provided for the structural members which are common to the above-described embodiment of the present invention. Explanation is omitted of the structural members already explained above.

As shown in FIG. 16, the treatment instrument insertion channel 5 which is a conduit is provided with a regulative member 130 in the bifurcation 8. The regulative member 130 comprises a substantially circular plate-shaped main body 131 connecting with the first conduit 9 and the main body 131 is fixed so that it crosses the bifurcation 8 at a slant toward the main conduit 7 to block a path communicating with the second conduit 10. Furthermore, as shown in FIG. 17, a plurality of slits 132 is provided in parallel on the main body 131. These slits 132 provide communication between the bifurcation 8 and the second conduit 10 and the slit 132 shapes the main body 131 as a grid except for its circumferential portion.

When the stent 30 is inserted by using the treatment instrument insertion channel 5, the guide catheter 25 on which the stent is provided thereon is inserted from the insertion slot 12. The stent 30 is inserted into the main conduit 7 from the first conduit 9 through the bifurcation 8 and because the movement of the stent 30 from the bifurcation 8 into the second conduit 10 is regulated by the regulative member 130, the stent 30 is guided into the main conduit 7 so as to be guided by the main body 131 of the regulative member 130. Because the inner diameter of the bifurcation 8, where the first conduit 9 proceeds to the second conduit 10, is kept substantially the same as that of the inner diameter of the first conduit 9, the stent 30 is inserted into the main conduit 7 without the flap 33 of the stent 30 open. On the other hand, when normal saline or the like is supplied from the second conduit, it passes through the slit 132, the bifurcation 8 and the main conduit 7 and is supplied into the human body.

Because the regulative member 130 is provided in the treatment instrument insertion channel 5, it is possible to reduce the inner diameter of the conduit when the stent 30 is inserted and because of this the flap 32 of the stent 30 does not open inside the treatment instrument insertion channel 5 and the flap 32 is prevented from being bent or the like. And there is no need to add special structures on the stent delivery catheter side.

For example, the stent installation member may be comprised of only a plate-like member which is extended from the blocking part 112. By using this kind of stent installation member, the same function and effect as those of the main body 111 and the blocking part 112 of the stent installation member 110 can be obtained. In this state, the marker 122 may be provided on the proximal end of the blocking part 112 to be able to check visually the insertion amount of the blocking part 112.

The first treatment instrument is not limited to the stent delivery catheter but it can be applied to a guide wire provided with a bending at the tip of a sheath already, a papilotomy knife and angiographic catheter. In the case of using this kind of treatment instrument, the insertion of the stent from the bifurcation 8 into the second conduit 10 is prevented and because of this the stent is prevented from becoming stuck inside of the treatment instrument insertion channel 5, making quick insertion and pulling out of the stent possible.

Further, according to another embodiment of the present invention, the stent delivery catheter is provided with a protective case which houses the stent delivery catheter in a state such that the stent is pulled over around the guide member and the flaps of the stent are open.

Because the stent delivery catheter is provided with the protective case, the flaps of the stent are prevented from being bent or the like while the stent delivery catheter is stored.

The insertion method is comprised of the stent installation member insertion process and the stent insertion process. The stent installation member insertion process is utilized when the stent, provided with flaps on the distal end and proximal end of the stent to be placed in human body, is inserted from the insertion slot provided in the divaricated conduit divaricated at the bifurcation from the main conduit of the endoscope which has an opening on its distal end. The stent installation member insertion process is to insert the stent installation member from the insertion slot of the divaricated conduit and to make the distal end of the stent installation member pass over the bifurcation and reach the main conduit after inserting the flap of the stent provided on its proximal end inside the stent installation member, which is longer than the route from the insertion slot to the bifurcation of the main conduit, and the stent can be inserted. The stent insertion process is to make at least the flap of the stent provided on its proximal end pass over the bifurcation through the stent installation member after the stent installation member insertion process.

According to the stent installation method, because the stent installation member insertion process is provided, the flap is prevented from opening in the bifurcation by reducing the apparent inner diameter of the conduit in the bifurcation when the stent passes over the bifurcation. As a result, unnecessary forces are not applied to the flap when the stent proceeds into the main conduit from the bifurcation.

According to the present invention, because it becomes possible to block a part of the bifurcation of the conduit in the endoscope and to reduce the diameter of a part, through which the first treatment instrument passes, it is possible to prevent the first treatment instrument being stuck at the bifurcation or a part of the first treatment instrument being bent. Therefore, it is possible to insert the first treatment instrument without fail.

While preferred embodiments of the invention have been described and illustrated above, it should be understood that these are exemplary of the invention and are not to be considered as limiting.

## Claims

1. A system composed of an endoscope (1), a treatment instrument installation device (35) and a first treatment instrument (30), said endoscope (1) comprising a treatment instrument insertion channel (5) comprising:
a first conduit (9) provided with an inner diameter through which the first treatment instrument (30) can be inserted,
a second conduit (10) provided with an inner diameter through which a second treatment instrument can be inserted,
a main conduit (7) provided with an inner diameter through which the first treatment instrument (30) and the second treatment instrument can be inserted, and
a connection part (8) which connects in a bifurcation the first conduit (9), the second conduit (10), and the main conduit (7), wherein the treatment instrument insertion channel (5) in the connection part (8) has an increased apparent inner diameter in the bifurcation as compared to the first conduit (9);
the treatment instrument installation device (35, 100, 110) comprising:
a main body (35a, 101, 111) provided with an outer diameter adapted to be inserted into the first conduit (9), and
a distal end part (35b, 101, 112) located at a distal side of the main body (35a, 101, 111),
**characterized in that**
the treatment instrument installation device (35, 100, 110) is adapted to be bent at the bifurcation and to stop as it fits into the first conduit (9) and the main conduit (7) to remain stationary and to allow said first treatment instrument (30) to move forward in distal direction, wherein
the treatment instrument installation device (35, 100, 110) comprises a blocking part such that, in the stopped state, it blocks at least part of the opening communicating with the second conduit (10) at the connection part (8).

2. The system according to claim 1, wherein
the main body (35a, 101, 111) is a tubular member provided with an outer diameter smaller than the inner diameter of the first conduit (9) and the connection part (8), and is also provided with an inner diameter larger than the outer diameter of the first treatment instrument (30), and
the main body (35a, 101, 111) is exposed from the insertion channel (5) at a first insertion slot (12) of the treatment instrument, which is located on an operation part (21) side of the insertion channel (5), while the distal end part (35b, 101, 112)
is adapted to block at least a part of the opening communicating with the second conduit (10) at the connection part (8).

3. The system according to claim 1 or 2, wherein
the treatment instrument installation device (35, 100, 110) is stopped by a regulative part (102) provided on the main body (111) on the proximal side to regulate the insertion amount into the first conduit (9).

4. The system according to one of the preceding claims, wherein the first treatment instrument is a catheter (20) with a stent (30),
wherein the catheter comprises:
a guide member (25) inserted into the first conduit (9) and the main conduit (7) from the treatment instrument (30),
wherein the stent (30) is movably provided on the guide member (25),
a pushing member (24) is provided movably in forward and backward direction on the guide member (25) to push out the stent, and
wherein the treatment instrument installation member (35) is a stent installation member (35), and is provided movably in forward and backward direction on the pushing member (24) and on the stent (30).

5. The system according to claim 5, wherein the stent is provided with flaps (32, 33) which regulate movement of the stent, and
the stent (30) is further provided with a protective case (70), wherein the protective case holds the stent in a state such that the stent is provided on a guide member (25), and
the protective case covers the stent in a state such that the flaps are open.

## Patentansprüche

1. System, gebildet aus einem Endoskop (1), einer Behandlungsinstrument-Installationsvorrichtung (35) und einem ersten Behandlungsinstrument (30), wobei das Endoskop (1) einen Behandlungsinstrument-Einführkanal (5) besitzt, der Folgendes aufweist:
einen ersten Kanal (9), der einen Innendurchmesser aufweist, durch den das erste Behandlungsinstrument (30) eingeführt werden kann,
einen zweiten Kanal (10), der einen Innendurchmesser aufweist, durch den ein zweites Behandlungsinstrument eingeführt werden kann,
einen Hauptkanal (7), der einen Innendurchmesser aufweist, durch den das erste Behandlungsinstrument (30) und das zweite Behandlungsinstrument eingeführt werden können, und
einen Verbindungsteil (8), der den ersten Kanal (9), den zweiten Kanal (10) und den Hauptkanal (7) in einer Gabelung miteinander verbindet, wobei der Behandlungsinstrument-Einführkanal (5) in dem Verbindungsteil (8) im Vergleich zu dem ersten Kanal (9) einen größeren scheinbaren Innendurchmesser in der Gabelung aufweist;
wobei die Behandlungsinstrument-Installationsvorrichtung (35, 100, 110) Folgendes aufweist:
einen Hauptkörper (35a, 101, 111), der einen Außendurchmesser aufweist, der sich in den ersten Kanal (9) einführen lässt, und
einen distalen Endteil (35b, 101, 112), der sich auf einer distalen Seite des Hauptkörpers (35a, 101, 111) befindet,
**dadurch gekennzeichnet,**
**dass** die Behandlungsinstrument-Installationsvorrichtung (35, 100, 110) dazu ausgebildet ist, an der Gabelung gebogen zu werden und beim Einpassen in den ersten Kanal (9) und den Hauptkanal (7) zu stoppen, so dass sie stationär bleibt und dem ersten Behandlungsinstrument (30) eine Vorwärtsbewegung in distaler Richtung ermöglicht,
wobei die Behandlungsinstrument-Installationsvorrichtung (35, 100, 110) ein Blockierteil aufweist, so dass dieses im gestoppten Zustand zumindest einen Teil der mit dem zweiten Kanal (10) an dem Verbindungsteil (8) in Verbindung stehenden Öffnung blockiert.

2. System nach Anspruch 1,
wobei es sich bei dem Hauptkörper (35a, 101, 111) um ein rohrförmiges Element handelt, das einen Außendurchmesser aufweist, der kleiner ist als der
Innendurchmesser des ersten Kanals (9) und des Verbindungsteils (8), und das ferner einen Innendurchmesser aufweist, der größer ist als der Außendurchmesser des ersten Behandlungsinstruments (30),
und wobei der Hauptkörper (35a, 101, 111) aus dem Einführkanal (5) an einer ersten, auf der Seite eines Betätigungsteils (21) des Einführkanals (5) gelegenen Einführschlitz (12) des Behandlungsinstruments freiliegt, während der distale Endteil (35b, 101, 112) dazu ausgebildet ist, zumindest einen Teil der mit dem zweiten Kanal (10) an dem Verbindungsteil (8) in Verbindung stehenden Öffnung zu blockieren.

3. System nach Anspruch 1 oder 2,
wobei die Behandlungsinstrument-Installationsvorrichtung (35, 100, 110) durch ein Regulierteil (102) gestoppt wird, das an dem Hauptkörper (111) auf der proximalen Seite vorgesehen ist, um das Einführausmaß in den ersten Kanal (9) zu regulieren.

4. System nach einem der vorausgehenden Ansprüche,
wobei es sich bei dem ersten Behandlungsinstrument um einen Katheter (20) mit einem Stent (30) handelt,
wobei der Katheter Folgendes aufweist:
ein Führungselement (25), das von dem Behandlungsinstrument (30) in den ersten Kanal (9) und den Hauptkanal (7) eingeführt wird,
wobei der Stent (30) an dem Führungselement (25) beweglich vorgesehen ist,
wobei ein Druck ausübendes Element (24) vor und zurück beweglich an dem Führungselement (25) vorgesehen ist, um den Stent nach außen zu drücken,
und wobei das Behandlungsinstrument-Installationselement (35) ein Stent-Installationselement (35) ist und dieses an dem Druck ausübenden Element (24) und an dem Stent (30) vor und zurück beweglich vorgesehen ist.

5. System nach Anspruch 5,
wobei der Stent mit Laschen (32, 33) versehen ist, die die Bewegung des Stents regulieren,
und wobei der Stent (30) ferner mit einem Schutzgehäuse (70) versehen ist, wobei das Schutzgehäuse den Stent in einem Zustand hält, in dem der Stent an einem Führungselement (25) vorgesehen ist, und
wobei das Schutzgehäuse den Stent in einem Zustand überdeckt, in dem die Laschen geöffnet sind.

## Revendications

1. Système composé d'un endoscope (1), d'un dispositif d'installation d'instrument de traitement (35) et d'un premier instrument de traitement (30), ledit endoscope (1) comprenant un canal d'insertion d'instrument de traitement (5) comprenant :
un premier conduit (9) possédant un diamètre interne à travers lequel le premier instrument de traitement (30) peut être inséré,
un deuxième conduit (10) possédant un diamètre interne à travers lequel un deuxième instrument de traitement peut être inséré,
un conduit principal (7) possédant un diamètre interne à travers lequel le premier instrument de traitement (30) et le deuxième instrument de traitement peuvent être insérés, et
une partie de raccordement (8) qui raccorde en une bifurcation le premier conduit (9), le deuxième conduit (10) et le conduit principal (7), dans lequel le canal d'insertion d'instrument de traitement (5) dans la partie de raccordement (8) possède un diamètre interne apparent augmenté dans la bifurcation comparé au premier conduit (9) ;
le dispositif d'installation d'instrument de traitement (35, 100, 110) comprenant :
un corps principal (35a, 101, 111) possédant un diamètre externe adapté pour être inséré dans le premier conduit (9), et
une partie d'extrémité distale (35b, 101, 112) placée au niveau d'un côté distal du corps principal (35a, 101, 111),
**caractérisé en ce que**
le dispositif d'installation d'instrument de traitement (35, 100, 110) est adapté pour être courbé au niveau de la bifurcation et pour buter à mesure qu'il s'ajuste dans le premier conduit (9) et le conduit principal (7) pour rester stationnaire et pour permettre que ledit premier instrument de traitement (30) se déplace vers l'avant dans le sens distal, dans lequel
le dispositif d'installation d'instrument de traitement (35, 100, 110) comprend une partie de blocage d'une manière telle que, dans l'état arrêté, il bloque au moins une partie de l'ouverture communicant avec le deuxième conduit (10) au niveau de la partie de raccordement (8).

2. Système selon la revendication 1, dans lequel
le corps principal (35a, 101, 111) est un élément tubulaire possédant un diamètre externe plus petit que le diamètre interne du premier conduit (9) et de la partie de raccordement (8), et possède également un diamètre interne plus grand que le diamètre externe du premier instrument de traitement (30), et
le corps principal (35a, 101, 111) est exposé à partir du canal d'insertion (5) au niveau d'une première fente d'insertion (12) de l'instrument de traitement, qui est placée sur une partie de fonctionnement (21) du côté du canal d'insertion (5), alors que la partie d'extrémité distale (35b, 101, 112) est adaptée pour bloquer au moins une partie de l'ouverture communicant avec le deuxième conduit (10) au niveau de la partie de raccordement (8).

3. Système selon la revendication 1 ou 2, dans lequel
le dispositif d'installation d'instrument de traitement (35, 100, 110) est arrêté par une partie de régulation (102) prévue sur le corps principal (111) au niveau du côté proximal pour réguler la quantité d'insertion dans le premier conduit (9).

4. Système selon l'une quelconque des revendications précédentes, dans lequel le premier instrument de traitement est un cathéter (20) avec une endoprothèse vasculaire (30),
dans lequel le cathéter comprend :
un élément de guidage (25) inséré dans le premier conduit (9) et dans le conduit principal (7) à partir de l'instrument de traitement (30),
dans lequel l'endoprothèse vasculaire (30) est prévue de manière amovible sur l'élément de guidage (25),
un élément de poussée (24) est prévu de manière amovible vers l'avant et vers l'arrière sur l'élément de guidage (25) pour faire sortir l'endoprothèse vasculaire, et
dans lequel l'élément d'installation d'instrument de traitement (35) est un élément d'installation d'endoprothèse vasculaire (35), et est prévu de manière amovible vers l'avant et vers l'arrière sur l'élément de poussée (24) et sur l'endoprothèse vasculaire (30).

5. Système selon la revendication 5, dans lequel l'endoprothèse vasculaire est pourvue de rabats (32, 33) qui régulent le mouvement de l'endoprothèse vasculaire, et
l'endoprothèse vasculaire (30) est en outre munie d'un boîtier protecteur (70), dans lequel le boîtier protecteur maintient l'endoprothèse vasculaire dans un état tel que l'endoprothèse vasculaire est prévue sur un élément de guidage (25), et
le boîtier protecteur couvre l'endoprothèse vasculaire dans un état d'une manière telle que les rabats sont ouverts.
